# EUROPEAN PATENT APPLICATION

(11) **EP 4 603 567 A1**
(43) Date of publication of application: **20.08.2025**
(21) Application number: 23877178.6
(22) Date of filing: 30.09.2023
(51) Int. Cl.: C11B 11/00, C12P 13/02, C11B 1/10

(54) **PLANT-DERIVED FREE CERAMIDE, AND METHOD FOR PRODUCING SAME AND METHOD FOR ANALYZING SAME**

(30) Priority: 11.10.2022 JP 2022162901
(71) Applicant: National University Corporation Saitama University, Saitama City, Saitama 338-8570 (JP)
(72) Inventor: ISHIKAWA Toshiki, Saitama-shi Saitama 338-8570 (JP); SANADA Sho, Saitama-shi Saitama 338-8570 (JP)
(74) Representative: Gulde & Partner
(86) International application number: PCT/JP2023/035818
(87) International publication number: WO 2024/080173

(57) **Abstract**

[Problems] To provide a method for producing plant-derived free ceramide simply and efficiently using a plant raw material.

[Solution] A method for producing plant-derived free ceramide comprising
an intrinsic enzyme activation step of destroying the cell structure of a plant tissue excluding calli to obtain a plant tissue homogenate in which phospholipase C that has glycosylinositol phosphoceramide as a substrate intrinsic in cells of the plant boy is activated; and
a degradation step of reacting the plant tissue homogenate by itself and/or together with another plant-derived glycosylinositol phosphoceramide-containing material at a temperature range of 20 to 30°C to selectively decompose the plant-derived glycosylinositol phosphoceramide into free ceramide by means of the phospholipase C.

## Description

### Technical Field

### Claiming Priority

This application claims the priority benefit of Japanese Patent Application No. 2022-162901 filed on October 11, 2022, and the entire contents of the prior application are deemed to be incorporated herein by reference. The original description of the prior application (including the claims, specification, and drawings) on which the priority claim is based shall be determined by considering the filing date of the prior application as the priority date (reference date), and shall not be affected by the description added this time.

The present invention relates to plant-derived free ceramide, and a method for producing same and a method for analyzing same.

### Background Art

Sphingolipids, which are components of biomembranes, are a general term for lipids composed of long-chain amino alcohols called long-chain bases (hereinafter sometimes referred to as "LCB"). Those in which fatty acids are bound to LCB are called free ceramides.

Sphingolipids present in plants are classified into four classes: free LCB, free ceramide (hereinafter sometimes referred to as "Cer"), glucosylceramide (hereinafter sometimes referred to as "GlcCer"), and glycosylinositol phosphoceramide (hereinafter sometimes referred to as "GIPC"). Of these, free LCB and free ceramide are minor components, while glucosylceramide and GIPC account for the majority of biomembrane lipids.

The human stratum corneum contains an extracellular lipid layer that is responsible for the barrier function of the skin. Free ceramide has a very long chain type (C20 or more) fatty acid, is a major component of the extracellular lipid layer, and is a substance that supports the skin barrier function. Skin function is improved when free ceramide is supplied transdermally or orally.

However, free ceramides are found in small amounts in nature. Therefore, chemically synthesized ceramides have been used in cosmetic uses.

In recent years, plant-derived ceramides have been attracting attention as ingredients in functional foods for skin care. Plant-derived ceramides are functional ingredients that far surpass hyaluronic acid and other ingredients, and its market size is expanding worldwide.

Currently, the plant-derived ceramide that is mainly used as an ingredient in functional foods is glucosylceramide. Glucosylceramide is composed of sugar and long-chain (C16-18) ceramide bonded together, and has a different structure from human free ceramide, which has very long-chain fatty acids. For this reason, it has a weak effect on the skin and is not suitable for cosmetic uses. **In** addition, when used as a food, there are issues such as low digestibility. Glucosylceramide does not completely replace the functionality of free ceramide.

Free ceramides of the same type as those in human skin are effective as cosmetic ingredients that can be applied directly to the skin, and are also expected to improve digestibility and absorption. However, free ceramides are rare ingredients that have not been found to accumulate naturally outside of the stratum corneum of animals, so the current mainstream is the use of chemical compounds containing analog compounds. Free ceramides, as safe and inexpensive plant-derived ingredients, are a particularly promising market need in the skin care industry, and are eagerly awaited as ingredients in foods and beverages, cosmetics, and pharmaceuticals.

In the meanwhile, GIPC is universally present in all plants, and its content is several times that of glucosylceramide. GIPC is known as the most abundant ceramide-containing substance on earth. GIPC is composed of a sugar chain and a very long chain type ceramide bonded together. In other words, the structure of the ceramide portion is the same as or similar to that of human skin ceramide. However, the sugar chain portion is extremely stable, and enzymatic degradation by animal digestive fluids or intestinal bacteria has not yet been found. Although GIPC is contained in large amounts in the plant foods we consume on a daily basis, it is an unused ceramide resource both biologically and industrially.

The chemical structures of the ceramide skeleton and hydrophilic portion of plants are highly diverse, and the total number of molecular species present in a single plant species reaches several hundred. Furthermore, the molecular composition differs depending on the plant species and tissues. It is not easy to grasp the whole picture using general analysis methods.

As a method for producing plant-derived free ceramide, Patent Document 1 discloses a method in which an alcoholic solvent is added to fermentation cake, which is a by-product generated during the production of a fermentation product using koji, to extract and purify the ceramide.

Patent 2 discloses a method for extracting and purifying free ceramide by adding an alcoholic solvent to a chestnut skin extract.

Patent Literature Document 3 discloses a method for producing free ceramide by heat treating crushed calli of an undifferentiated plant at 45°C for 5 hours to induce autophagy.

Patent Document 4 discloses a composition for digesting GIPC or GlcCer to produce free ceramide, which contains a mushroom autolysis liquid or a purified product thereof, as a method for preparing free ceramide from GIPC and GlcCer.

Non-Patent Document 1 discloses that an enzyme activity (hereinafter, sometimes referred to as "GIPC-phospholipase D" or "GIPC-PLD") that specifically hydrolyzes GIPC into phytoceramide-1-phosphate (hereinafter, sometimes referred to as "PC1P") has been found in cabbage, that this enzyme activity is activated when leaf tissue is ground, and that it is speculated that in vivo PC1P is converted into free ceramide by phosphatase in the digestive tract.

Non-Patent Document 2 is a method for extracting plant ceramide-related substances known as the Bligh & Dyer method. A mixture of chloroform, methanol, and water is used as the basic extraction solvent, and there is a variation in which potassium chloride or hydrochloric acid is mixed in. Water-soluble and insoluble contaminants can be removed by two-phase distribution between a chloroform phase and an aqueous phase, and this is the most commonly used lipid extraction method.

Non-Patent Document 3 discloses a method for extracting plant ceramide-related substances known as the Markham method. This method uses isopropanol as an extraction solvent and can efficiently extract plant ceramide-related substances including GIPC.

Non-Patent Document 4 discloses an analysis method for analyzing the structural diversity of plant sphingolipids by liquid chromatography-tandem mass spectrometry (LC-MS/MS).

However, the methods of Patent Documents 1 and 2 merely obtain free ceramide already present in the raw material by repeating the steps of extraction and purification using an alcohol-based solvent multiple times. Considering the content of free ceramide in the raw material, these methods are not efficient.

The method of Patent Document 3 requires a medium and equipment for culturing the calli of an undifferentiated plant in advance, and is therefore not simple and efficient for mass production. In addition, the method of Patent Document 3 requires a step of heating (45 to 70°C) to subject the plant to autophagy-inducing conditions. Furthermore, since autophagy is a physiological phenomenon that occurs in living cells, the plant raw material is limited to living cells.

In the method of Patent Document 4, the mushroom autolysis liquid also decomposes GlcCer, which has high utility value, and is not a method for selectively decomposing only GIPC into free ceramide. In addition, a separate step of preparing the mushroom autolysis liquid is required. Furthermore, pretreatment is required, such as extracting GIPC and GlcCer in advance from the raw plant material, which is cumbersome. In addition, mushrooms are not plants but fungi. The method using the mushroom autolysis liquid utilizes heterologous enzymes and cannot be said to be a method for producing free ceramide using only plant-derived components.

In the method of Non-Patent Document 1, in order to obtain free ceramide from GIPC, under in vitro conditions, it is necessary to first decompose GIPC with GIPC-PLD to obtain PC1P, and then dephosphorylate PC1P with phosphatase. The method of Non-Patent Document 1 requires two different enzymes and cannot be said to be efficient.

The method of Non-Patent Document 2 is suitable for extracting free ceramide and GlcCer, but is not suitable for comprehensive analysis of plant ceramide-related substances because GIPC is hardly recovered in the organic phase. In addition, chloroform is highly toxic, and its use has been avoided in recent years.

The method of Non-Patent Document 3 requires a long extraction process and requires the use of many extraction vessels. In addition, a large amount of solvent containing low volatility water is used, so it takes a long time to evaporate and remove the solvent. In addition, since two-phase distribution is not performed, there are many contaminants. In particular, the inclusion of polysaccharides reduces the quantitative value of GIPC by LC-MS/MS.

The method of Non-Patent Document 4 describes that the content and composition of molecular species of each plant sphingolipid class can be comprehensively overviewed by combining high performance liquid chromatography (hereinafter sometimes referred to as "HPLC") and mass spectrometry by multiple reaction monitoring (hereinafter sometimes referred to as "MRM"), but a method for simultaneously analyzing free ceramide, GIPC, and GlcCer is not disclosed. In addition, it is disclosed that there are still many issues to be investigated in order to simply and comprehensively analyze plant sphingolipid molecules with diverse structures. For example, the total number of molecular species present in one plant species can reach several hundred, and the molecular composition varies depending on the plant species and the part of the plant tissue. However, the number of molecules that can be detected simultaneously by general MRM analysis is limited to about 50 to 100, and it is not easy to grasp the whole picture.

### Citation List

### Patent Documents

Patent Document 1: JP-A No. 2012-41518
Patent Document 2: WO2018/021476
Patent Document 3: JP-A No. 2019-115318
Patent Document 4: JP-A No. 2021-103950

### Non-Patent Documents

Non-Patent Document 1: Takashi Kita et al.: Lipid Nutrition, Vol. 25 (1), pp. 75-85, 2016
Non-Patent Document 2: E. G. BLIGH, W. J. DYER: Canadian Journal of Biochemistry and Physiology 1959 August; 37(8): 911-917
Non-Patent Document 3: Jennifer E. Markham et al: J Biol Chem. 2006 Aug; 281(32):22684-94
Non-Patent Document 4: Hiroyuki Imai et al.: Biochemistry, Vol. 88 (1), pp. 94-104, 2016

### Summary of Invention

### Technical Problem

The present invention has been made to solve the above problems, and aims to provide a method for selectively, simply and efficiently producing plant-derived free ceramide from GIPC contained in a plant raw material. Another aim of the present invention is to provide a method for efficiently extracting plant-derived free ceramide. Another aim of the present invention is to provide a method for simultaneously analyzing plant-derived free ceramide, GIPC, and GlcCer in a short period of time.

### Solution to Problem

As a result of intensive research conducted by the present inventors in consideration of the problems of the prior art, it was found that by utilizing the activity of phospholipase C, which uses GIPC contained in the plant tissue except for calli as a substrate, it is possible to simply and efficiently produce plant-derived free ceramide, that by using a specific extraction solvent, it is possible to efficiently extract plant-derived free ceramide, and further, that by combining HPLC with scheduled MRM (detection time difference setting), it is possible to simultaneously analyze plant-derived free ceramide, GIPC, and GlcCer in a short period of time. Based on these findings, the present inventors have made repeated improvements and have completed the present invention.

That is, the present invention provides the following inventions.
[1] A method for producing plant-derived free ceramide, comprising
   an enzyme activation step of destroying cells structure of a plant tissue excluding calli to obtain a plant tissue homogenate in which an endogenous activated phospholipase C that hydrolyzes glycosylinositol phosphoceramide as a substrate is included; and
   a degradation step of reacting the plant tissue homogenate with same and /or another plant-derived glycosylinositol phosphoceramide-containing material at a temperature range of 20 to 30°C to selectively hydrolyze the glycosylinositol phosphoceramide into free ceramide by the endogenous activated phospholipase C.
[2] The method for producing plant-derived free ceramide according to [1], wherein the glycosylinositol phosphoceramide-containing material is any one of the following or a combination thereof:
   (a) the plant tissue containing glycosylinositol phosphoceramide,
   (b) a different plant tissue containing glycosylinositol phosphoceramide,
   (c) glycosylinositol phosphoceramide, extracted from the plant tissue containing the phospholipase C and
   (d) glycosylinositol phosphoceramide extracted from a tissue of other plant species
[3] The method for producing plant-derived free ceramide according to [1], wherein the mixing weight ratio of the plant tissue homogenate to the glycosylinositol phosphoceramide-containing material in the degradation step is 1:0 to 1:150.
[4] Plant-derived free ceramide obtained by the production method according to any one of [1] to [3].
[5] A food or drink comprising the plant-derived free ceramide according to [4].
[6] A cosmetic comprising the plant-derived free ceramide according to [4].
[7] A pharmaceutical comprising the plant-derived free ceramide according to [4].
[8] A method for extracting the plant-derived free ceramide obtained by the production method according to [1], comprising
   an alkaline treatment step for decomposing ester lipids by adding a 1-butanol/methanol mixture to a sample for lipid extraction, and then treating the sample under mild alkaline conditions ; and
   a step for obtaining an extract by adding 1-butanol and a strong acid to the sample after the alkaline treatment step, separating the sample into two layers under acidic conditions, and recovering the upper 1-butanol-rich layer to obtain an extract containing free ceramide, glycosylinositol phosphoceramide, and glucosylceramide.
[9] A method for analyzing the plant-derived free ceramide obtained by the production method according to [1], comprising
   an alkaline treatment step for decomposing ester lipids by adding a 1-butanol/methanol mixture to a sample for lipid extraction treatment, and then treating the sample under mild alkaline conditions;
   a step for obtaining an extract by adding 1-butanol and a strong acid to the sample after the alkaline treatment step, separating the sample into two layers under acidic conditions, and recovering the upper 1-butanol-rich layer to obtain an extract containing free ceramide, glycosylinositol phosphoceramide, and glucosylceramide; and
   an analysis step of introducing the extract into a liquid chromatograph-tandem mass spectrometer, separating molecular species of free ceramide, glycosylinositol phosphoceramide, and glucosylceramide in the extract by liquid chromatography using a reversed-phase column, and performing mass spectrometry on the separated three substances with the mass spectrometer,
   wherein in the analysis step, an MRM measurement is performed for selective detection and quantification of the substances, which is scheduled to monitor around chromatographic elution time of each substances, allowing simultaneous quantification of the whole species of free ceramide, glycosylinositol phosphoceramide, and glucosylceramide contained in the extract.
[10] A method for analyzing plant-derived free ceramide, comprising
   a step of preparing the free ceramide produced by the production method according to [1] as an analyte;
   a step of preparing a control sample by homogenizing a plant tissue after heating or freeze-drying;
   an alkaline treatment step for decomposing ester lipids by adding a 1-butanol/methanol mixture to a sample for lipid extraction treatment, and then treating the sample under mild alkaline conditions;
   a step for obtaining an extract by adding 1-butanol and a strong acid to the sample after the alkaline treatment step, separating the sample into two layers under acidic conditions, and recovering the upper 1-butanol-rich layer to obtain an extract containing free ceramide, glycosylinositol phosphoceramide, and glucosylceramide; and
   an analysis step of introducing each of the extracts into a liquid chromatograph-tandem mass spectrometer, separating molecular species of free ceramide, glycosylinositol phosphoceramide, and glucosylceramide in the extract by liquid chromatography using a reversed-phase column, and performing mass analysis of the separated three substances with the mass spectrometer,
   wherein in the analysis step, an MRM measurement is performed for selective detection and quantification of the substances, which is scheduled to monitor around chromatographic elution time of each substance, and the whole species of ceramide, glycosylinositol phosphoceramide contained in the extracts are measured simultaneously, and then the amount of free ceramide produced is evaluated by the increased amount of free ceramide and decreased amount of glycosylinositol phosphoceramide.

### Advantageous Effects of Invention

According to the production method of the present invention, a method for selectively, simply and efficiently producing plant-derived free ceramide from GIPC contained in a plant raw material can be provided.

**In** the production method of the present invention, GIPC, which is abundant in plant tissue, is decomposed directly by phospholipase C, which uses GIPC present in the plant tissue as a substrate, and therefore there is no need to rely on heterologous enzymes or special chemical treatments. GIPC is not only found in large quantities in all plants, but is also thought to remain in large quantities in plant waste due to its stability and poor solubility. According to the production method of the present invention, GIPC, which has not been used in the past, can be decomposed to directly produce free ceramide. **In** addition, there is no need for special plant materials that require cultivation, or complicated techniques, and free ceramide can be produced simply, quickly, in large quantities, and at low cost.

The free ceramide obtained by the production method of the present invention can be used, for example, as an ingredient or raw material for foods and beverages including functional foods, and cosmetics, pharmaceuticals, etc.

Furthermore, according to the extraction method of the present invention, a method for efficiently extracting plant-derived free ceramide, GIPC, and GlcCer can be provided.

Furthermore, the analysis method of the present invention can provide a method for simultaneously analyzing plant-derived free ceramide, GIPC, and GlcCer in a short period of time.

Plants contain a wide variety of ceramide-related substances, and their molecular compositions vary greatly depending on the plant species and tissues. The analysis method of the present invention can obtain quantitative data that comprehensively covers all of these, and will greatly contribute to the establishment of a method for mass-producing free ceramide using various plant species as raw materials.

### Brief Description of Drawings

[Fig. 1] Fig. 1 shows an overall image of scheduled MRM in the analysis method of the present invention and a superimposition of all chromatographs.
[Fig. 2] Fig. 2 shows the results of a preliminary experiment evaluating the extraction efficiency of plant ceramide-related substances.
[Fig. 3] Fig. 3 shows the results of GIPC degradation and free ceramide production in Arabidopsis homogenate after standing for 30 minutes.
[Fig. 4] Fig. 4 shows the results of GIPC degradation and free ceramide production in Arabidopsis homogenate after standing for 10 to 60 minutes.
[Fig. 5] Fig. 5 shows the results of GIPC degradation and production of free ceramide in various plant bodies.
[Fig. 6] Fig. 6 shows the results of degradation of GIPC and production of free ceramide in a heterologous plant tissue (carrot) by Arabidopsis homogenate.
[Fig. 7] Fig. 7 shows the results of degradation of soybean-derived GIPC and production of free ceramide by Arabidopsis homogenate.
[Fig. 8] Fig. 8 shows a comparison between Enoki autolysis liquid and Arabidopsis homogenate, and the results of GIPC degradation and free ceramide production by mixing with a heterologous plant tissue (carrot).
[Fig. 9] Fig. 9 shows the results of GIPC degradation and free ceramide production when GIPC-PLD was added to Arabidopsis homogenate.
[Fig. 10] Fig. 10 is a diagram showing a presumed mechanism of the GIPC degradation pathway.
[Fig. 11] Fig. 11 is a diagram showing the structure of a representative GIPC.
[Fig. 12] Fig. 12 is a graph showing the production of free ceramide from GIPC by phospholipase (PLC).
[Fig. 13] Fig. 13 shows differences in reaction products due to differences in degradation enzymes (PLD and PLC).
[Fig. 14] Fig. 14 is a graph showing the difference in the amount of free ceramide produced due to different temperature conditions in the degradation step.

### Description of Embodiments

The present invention will be described below with reference to specific embodiments. Note that the configurations shown in the following embodiments are merely examples, and the present invention is not limited to these configurations.

### Method for producing plant-derived free ceramide

The method for producing plant-derived free ceramide according to the present invention comprises
an enzyme activation step by destroying cells of a plant tissue excluding calli to obtain a plant tissue homogenate in which an endogenous activated phospholipase C that hydrolyzes glycosylinositol phosphoceramide as a substrate is included; and
a degradation step of the glycosylinositol phosphoceramide by reacting the plant tissue homogenate together with same and /or another plant-derived glycosylinositol phosphoceramide-containing material at a temperature in the range of 20 to 30°C to selectively hydrolyze the glycosylinositol phosphoceramide into free ceramide by the phospholipase C.

### (Enzyme activation step)

In the enzyme activation step, cells of a plant tissue excluding calli is destroyed to obtain a plant tissue homogenate. An endogenous activated phospholipase C is included in the plant tissue homogenate. The endogenous activated phospholipase C is activated and it hydrolyzes the glycosylinositol phosphoceramide as a substrate.

In the present invention, the plant tissue includes not only the plant itself, but also parts thereof such as plant organs, such as leaves, stems, and roots, plant tissues, seeds, and fruits. The plant tissue of the present invention also includes plant waste (plant residue), but excludes calli of undifferentiated plants.

Examples of plant include, but are not limited to, Brassicaceae plants (cabbage, Japanese radish, radish, broccoli, Japanese mustard spinach, Arabidopsis, bok choy, mizuna, etc.), Apiaceae plants (carrot, celery, Japanese parsley, coriander, angelica tree, etc.), Leguminous plants (soybean, mung bean sprouts, etc.), Liliaceae plants (onion, leek, etc.), Lamiaceae plants (basil, Japanese basil, Japanese perilla, Japanese sesame, etc.), Cucurbitaceae plants (cucumber, watermelon, pumpkin, zucchini, etc.), various vegetable waste, fruit skins and seeds, soybean meal, coffee grounds, brewery waste, etc.

In this specification, "crush" refers to a state in which the cell membrane is crushed and the living cells are destroyed to the extent that the enzymes contained in the plant tissue are activated. Specifically, the tissue mass is sufficiently finely divided. For example, the crushed state refers to a homogenous suspension in which no large masses of several mm are observed and the tissue cannot be further finely divided even if the crush process is continued, that is, a so-called homogenate state.

As the crush means, any method can be used as long as it generates a shear force sufficient to destroy living cells. Examples of the crush means include, but are not limited to, mechanical methods such as a mixer (Waring blender), a food processor, a homogenizer, and ultrasonic treatment. In one embodiment of the present invention, when a mixer (Waring blender) is used, the cells can be crushed by processing for 1 to 10 minutes until the cells reach the crushed state described above.

When crushing, the plant tissue may be in any state, such as raw, dried, or frozen, but must be in a moist state. When using a dried plant tissue, for example, water is added in an amount 5 to 20 times the weight of the dried plant tissue before crushing. In addition, since the cell membrane of a plant tissue that has been frozen is damaged by freezing, free ceramide can be efficiently obtained by crushing the frozen plant tissue.

The crushing time may be appropriately set depending on the amount of the plant tissue and the crushing means.

An example of an enzyme present in a plant tissue is phospholipase C, which is a lipid-decomposing enzyme. Phospholipase C is an enzyme detected in a wide range of plant species, and generally cleaves the glycerol-phosphate ester bond of glycerophospholipids on the lipid side. In this specification, phospholipase C that uses glycosylinositol phosphoceramide as a substrate to produce a sugar chain and free ceramide may be referred to as "GIPC-phospholipase C" or "GIPC-PLC".

The identity of the protein that catalyzes the enzyme reaction of GIPC-PLC is unknown. However, as shown in the Examples below, the decrease in GIPC and the increase in free ceramide are almost equal, and the addition of GIPC-PLD disclosed in Non-Patent Document 1 to the reaction system does not increase free ceramide, and therefore, the increase in free ceramide observed in the present invention is directly generated from GIPC, which supports the existence of GIPC-PLC activity.

Among the above-mentioned plant bodies, Brassicaceae plants, particularly Arabidopsis, contain highly active GIPC-PLC. Therefore, by using a homogenate of a Brassicaceae plant as a reaction substrate, free ceramide can be efficiently produced.

The active temperature range of GIPC-PLC is usually 20 to 30°C. Therefore, the temperature condition in the intrinsic enzyme activation step is preferably within the range of 20 to 30°C, and from the viewpoint of promoting the enzyme reaction, 20 to 28°C is preferable, and 20 to 25°C is more preferable.

### (Degradation step)

In the degradation step, the plant tissue homogenate is reacted together with same and /or another plant-derived glycosylinositol phosphoceramide-containing material as the plant tissue homogenate at a temperature range of 20 to 30°C to selectively hydrolyze the glycosylinositol phosphoceramide into free ceramide by the endogenous activated phospholipase C.

The reaction temperature in the degradation step is preferably within the range of 20 to 30°C from the viewpoint of being within the active temperature range of GIPC-PLC, and is preferably 20 to 28°C, more preferably 20 to 25°C from the viewpoint of promoting the enzyme reaction.

The term "reacting the plant tissue homogenate together with same plant-derived glycosylinositol phosphoceramide-containing material", namely, "reacting the plant tissue homogenate alone" refers to, for example, allowing the plant tissue homogenate to stand in a container to react the GIPC-PLC intrinsically present in the plant tissue and the GIPC in the plant tissue.

The reaction of the plant tissue homogenate with a GIPC-containing material derived from another plant means, for example, placing the plant tissue homogenate and the GIPC-containing material derived from another plant in the same container and mixing them.

The mixing means is not particularly limited, but it is preferable to mix while stirring. Examples of such mixing means include, but are not limited to, a general mixer (e.g., a Waring blender, a Nauta mixer, a Henschel mixer, a butterfly mixer, etc.), a food processor, a homogenizer, a blender, etc.

As used herein, "reacting the plant tissue homogenate together with same and/or another plant-derived glycosylinositol phosphoceramide-containing material" means reacting the plant tissue homogenate alone, reacting the plant tissue homogenate together with another plant-derived glycosylinositol phosphoceramide-containing material, and combinations of the above.

The reaction time in the degradation step is, for example, 10 minutes to 48 hours, and preferably 10 minutes to 24 hours. This can be appropriately set depending on the amounts of the plant tissue homogenate and the plant-derived GIPC-containing material.

The GIPC-containing material is any one of the following or a combination thereof:
(a) The plant tissue containing GIPC.
(b) A different plant tissue containing GIPC.
(c) GIPC extracted from the plant tissue containing the phospholipase C.
(d) GIPC extracted from a tissue of other plant species.

The types of plant bodies containing GIPC are as already exemplified in the section on the intrinsic enzyme activation step. The plant bodies containing GIPC used in this degradation step may be plant tissue whose own GIPC degradation enzyme activity is weak, have no GIPC degradation enzyme activity, or have inactivated GIPC degradation enzyme activity.

The plant tissue containing GIPC may be in any state, such as raw, dried, or frozen, but must contain moisture. When using a dried plant tissue, for example, water is added in an amount of 5 to 20 times the weight of the dried plant tissue.

Examples of the form of the plant tissue containing GIPC include whole, chipped, chopped, sliced, roughly ground, pelleted, powdered, paste-like (homogenized), etc. From the viewpoint of efficient reaction with the plant tissue homogenate, the plant tissue is preferably in the form of finely pulverized, and more preferably in the form of a paste (homogenized) in which the tissue mass is sufficiently finely pulverized to a state in which the cell membrane of the plant tissue is crushed and the living cells are destroyed using the crushing means in the intrinsic enzyme activation step.

In the degradation step, the mixing weight ratio of the plant tissue homogenate to the GIPC-containing material is, for example, 1:0 to 1:150. Note that the mixing weight ratio of 1:0 means that the GIPC contained in the plant tissue homogenate is decomposed, i.e., the plant tissue homogenate is used by itself, not as a mixture.

From the viewpoint of obtaining free ceramide efficiently in a short time, the mixing weight ratio of the plant tissue homogenate to the GIPC-containing material is preferably 1:0 to 1:10, and more preferably 1:0 to 1:4.

The enzyme activation step and the degradation step may be performed in this order, or may be performed simultaneously in parallel. For example, among the above-mentioned plant tissue, Brassicaceae plants, particularly Arabidopsis, have highly active GIPC-PLC intrinsically. The Brassicaceae plant and other GIPC-containing materials (e.g., plant bodies with low GIPC degradation enzyme activity) are mixed and crushed in the same container, and the mixture is allowed to stand at a temperature range of 20 to 30°C for reaction, whereby the intrinsic enzyme activation step and the degradation step can proceed simultaneously.

The plant-derived free ceramide obtained by the above-mentioned production method can be extracted by the following method.

### Method for extracting plant-derived free ceramide

The method for extracting plant-derived free ceramide according to the present invention is a method for extracting plant-derived free ceramide obtained by the production method of the present invention, comprising

A method for extracting the plant-derived free ceramide obtained by the production method according to [1], comprising
an alkaline treatment step for decomposing ester lipids by adding a 1-butanol/methanol mixture to a sample for lipid extraction, and then treating the sample under mild alkaline conditions ; and
a step for obtaining an extract by adding 1-butanol and a strong acid to the sample after the alkaline treatment step, separating the sample into two layers under acidic conditions, and recovering the upper 1-butanol-rich layer to obtain an extract containing free ceramide, glycosylinositol phosphoceramide, and glucosylceramide.

### (Alkaline treatment step))

In the alkaline treatment step, a sample is extracted with a 1-butanol/methanol mixture added, and then treated under mild alkaline conditions to decompose ester lipids.

The term "sample" as used herein refers to plant-derived free ceramide obtained by the production method of the present invention, and refers to any sample including a plant tissue homogenate that has been subjected to an intrinsic enzyme activation step and a degradation step. The sample may be used directly as it is, or may be pretreated and/or prepared.

The volume ratio of 1-butanol to methanol in the 1-butanol/methanol mixture is usually 1:6 to 6:1 (v:v) or any value within this range, preferably 1:4 to 4:1 (v:v) or any value within this range, more preferably 1:3 to 3:1 (v:v) or any value within this range, and even more preferably 2:1 (v:v).

The temperature at which the sample is extracted with a 1-butanol/methanol mixture added is usually 50°C or higher, and preferably 75°C or higher. The reaction time is usually 5 minutes or longer, and preferably 10 minutes or longer.

Examples of the alkaline agent to be used under mild alkaline conditions include sodium hydroxide, potassium hydroxide, etc. Only one type of alkaline agent may be used, or two or more types may be used in combination.

The volume ratio of the 1-butanol/methanol mixed solution to the alkaline agent is usually 1:6 to 6:1 (v:v) or any value within this range, preferably 1:4 to 4:1 (v:v) or any value within this range, more preferably 1:3 to 3:1 (v:v) or any value within this range, and even more preferably 1:0.3 to 1:0.6 (v:v) or any value within this range. Depending on the mixing ratio, the solution may separate into two layers, in which case a small amount of methanol may be added to make a uniform mixed solvent, which can promote the degradation of the ester lipid contained in the organic layer.

In the treatment under alkaline conditions, the lower limit of the reaction temperature is usually 20°C or higher, preferably 30°C or higher, and more preferably 45°C or higher. The upper limit is usually 70°C or lower, preferably 60°C or lower, and more preferably 55°C or lower. Therefore, the reaction temperature is usually 20 to 70°C, preferably 30 to 60°C, and more preferably 45 to 55°C. The reaction time is usually 5 minutes or longer, and preferably 10 minutes or longer. By the treatment under these mild alkaline conditions, the lipid esters in the sample are decomposed.

### (Step of obtaining extract)

In the step for obtaining an extract, 1-butanol and a strong acid are added to the sample after the alkaline treatment step, and the sample is separated into two layers under acidic conditions, and recovering the upper 1-butanol-rich layer to obtain an extract containing free ceramide, glycosylinositol phosphoceramide, and glucosylceramide.

The volume ratio of the sample after the degradation step to 1-butanol and strong acid is usually 1:1.5:1.5 to 1:3:5 (v:v:v) or any value within this range, and is preferably 1:2:3 (v:v:v).

Examples of the strong acid include inorganic acids such as hydrochloric acid, nitric acid, sulfuric acid, phosphoric acid, etc. Only one type of strong acid may be used, or two or more types may be used in combination.

By carrying out two-phase distribution between a 1-butanol/methanol mixture and water under acidic conditions, free ceramide, glycosylinositol phosphoceramide, and glucosylceramide can be recovered in the 1-butanol phase, and contaminants including interfering components (e.g., polysaccharides) can be removed into the aqueous phase. The extract recovered from the 1-butanol phase contains total lipids including degradation intermediates in which the hydrophilic portion of GIPC is gradually cleaved.

### (Other steps)

After a process such as evaporating and removing the solvent from the extract, plant-derived free ceramide can be obtained.

The main fatty acid chain length of the plant-derived free ceramide obtained by the production method of the present invention varies slightly depending on the plant species, but is mostly distributed between C22 and C26, with C24 being the center, which is the same as the human type. In this respect, it is different from glucosylceramide, which is mainly composed of C16 to C20, and synthetic ceramide, which is a single molecular species.

The extraction method of the present invention can be performed in a single vessel from saponification to two-phase distribution, and requires fewer steps. In addition, since a smaller amount of solvent is used than in the conventional techniques, simultaneous extraction of multiple specimens can be easily performed using a vessel with a small capacity. Furthermore, since the extract is obtained in a small amount of organic solvent by two-phase distribution, evaporation removal can be completed in a short time. The recovery rate of plant ceramide-related substances by the extraction method of the present invention is superior to that of the Markham method, and in particular, the removal of polysaccharides can improve the detection sensitivity of GIPC LC-MS/MS. A comparison of the extraction method of the present invention with the conventional techniques is summarized in Table 1.

**[Table 1]**

| | **Extraction method of the present invention** | **Bligh & Dyer method** | **Markham Method** |
|---|---|---|---|
| Solvent used*1 | 1-butanol, methanol, water, strong alkali (e.g., KOH/NaOH), strong acid (e.g., HCl) | Chloroform, methanol, water, KCl, HCl, KOH/NaOH | Isopropanol, hexane, water, methylamine |
| Distribution | Ceramide-related substances are distributed in the organic phase, and polysaccharides are removed into the aqueous phase | Ceramide-related substances, except for GIPC, are distributed into the chloroform phase | None (many contaminants) |
| Final amount of extract (per 1 g of sample) *2 | 3ml | 10 to 50 ml | 30 to 50 ml |
| Time required for extraction*3 | 3 hours, 24-48 specimens | 3 hours, 8 to 16 specimens | 5 hours, 8 to 16 specimens |
| Application to ceramide-related substances | Nearly 100% of all molecular weights are recovered, enabling LC-MS/MS analysis. | Extremely low yield of GIPC | Many molecular species can be recovered, but the detection sensitivity of CIPC decreases due to contaminants |

| | | | |
|---|---|---|---|
| *1: Chemicals used for detection and saponification treatment *2: Final liquid volume after two-phase distribution, general guideline *3: The time required for one extraction operation using general-purpose equipment such as a centrifuge, and the number of specimens that can be treated | | | |

### Analysis method 1 for plant-derived free ceramide

The method for analyzing plant-derived free ceramide according to the present invention is a method for analyzing free ceramide obtained by the production method, comprising
an alkaline treatment step for decomposing ester lipids by adding a 1-butanol/methanol mixture to a sample for lipid extraction treatment, and then treating the sample under mild alkaline conditions to decompose the ester lipids;
a step for obtaining an extract by adding 1-butanol and a strong acid to the sample after the alkaline treatment step, separating the sample into two layers under acidic conditions, and recovering the upper 1-butanol-rich layer to obtain an extract containing free ceramide, glycosylinositol phosphoceramide, and glucosylceramide; and
an analysis step of introducing the extract into a liquid chromatograph-tandem mass spectrometer, separating free ceramide, glycosylinositol phosphoceramide, and glucosylceramide in the extract by liquid chromatography using a reversed-phase column, and performing mass spectrometry on the separated three substances with the mass spectrometer,
wherein in the analysis step, an MRM measurement is carried out scheduled to selectively detect only the time around when each of the three substances is eluted from a liquid chromatograph, and the three substances contained in the extract are simultaneously and quantitatively analyzed.

### (Step for obtaining extract)

The above steps have already been described in the section on the detailed description of the extraction method of the present invention. In one embodiment of the present invention, a 1-butanol/methanol (volume ratio 2:1) mixture is added to a sample, and the sample is treated at 75°C or higher for 10 minutes or more, and then 0.3 to 1 volumes (volume) of a strong alkali (e.g., 1N potassium hydroxide, etc.) is added, and the sample is treated at 45 to 70°C for 10 minutes or more to decompose ester lipids, and then 1.5 to 3 volumes (volume) of 1-butanol and 1.5 to 5 volumes (volume) of a strong acid (e.g., 0.4N hydrochloric acid, etc.) are added to acidify the sample and separate it into two layers, and 1-butanol in the upper layer is recovered to obtain an extract containing free ceramide, glycosylinositol phosphoceramide, and glucosylceramide.

### (Analysis step)

In the analysis step, the extract is introduced into a liquid chromatograph-tandem mass spectrometer, and free ceramide, GIPC, and glucosylceramide in the extract are separated by liquid chromatography using a reversed-phase column, while the separated three substances are subjected to mass analysis by the mass spectrometer, and an MRM measurement (multiple reaction monitoring) is performed scheduled to selectively detect only the time around when each of the three substances is eluted from the liquid chromatograph, thereby simultaneously and quantitatively analyzing the three substances contained in the extract.

The liquid chromatograph-tandem mass spectrometer referred to here is, for example, a liquid chromatograph-triple quadrupole mass spectrometer or a liquid chromatograph-quadrupole/time-of-flight (Q-TOF) mass spectrometer.

Typically, in such liquid chromatograph-tandem mass spectrometers, analytical parameters can be arbitrarily determined so as to maximize the detection sensitivity, i.e., to obtain the best possible conditions, for each component in the extract after separation by liquid chromatograph using a reversed-phase column.

A list of structural types of plant ceramides and related metabolites is shown in Table 2.

**[Table 2]**

| **Ceramide types and theoretical total number of molecular species** | **Hydrophilic area** | **Ceramide skeleton** | |
|---|---|---|---|
| | | **Long-chain bases** | **Fatty acids** |
| Free ceramide (Cer) 1×6×30 = 180 types | 1 type (-Glc) | 6 types (t18:0, t18:1, d18:0, d18:1, d18:2, d18:3) | 30 types (16:0, 18:0, 20:0, 20:1, 21:0, 22:0, 22:1, 23:0, 23:1, 24:0, 24:1, 25:0, 25:1, 26:0, 26:1, all with two types each of normal and alpha hydroxy) |
| Glucosylceramide (GlcCer) 1×6×30 = 180 types | 1 type (-OH) | 6 types (t18:0, t18:1, d18:0, d18:1, d18:2, d18:3) | |
| CIPC 6×4×30=720 types | 6 types (-Hex, - HexHex, -HexN, - HexNHex, -HexNAc, -HexNAcHex) | 4 types (t18:0, t18:1, d18:0, d18:1) | |
| PC1P (acetylated) 1×4×30 = 120 types | 1 type (-Pi) | 4 types (t18:0, t18:1, d18:0, d18:1) | |

In the table, "Glc" in the hydrophilic portion represents glucose, "OH" represents a hydroxyl group, "Hex" represents hexose, "HexN" represents hexosamine, and "HexNAc" represents N-acetylhexosamine. In the notation of the ceramide skeleton such as "t18:0", "t" means that it has three hydroxy groups, "18" represents the number of carbon atoms, and the last number "0" represents the number of double bonds. In addition, "d" represents a dihydroxy base.

An MRM measurement is performed that is scheduled to selectively detect only the time around when each of the three substances is eluted from the liquid chromatograph (for example, elution time ±1 minute). For example, the elution time of GIPC is 6 to 19 minutes, that of GlcCer is 15 to 25 minutes, and that of Cer is 20 to 30 minutes. This makes it possible to suppress the number of overlapping molecules to 100 or less, and to simultaneously measure 1000 or more molecular species. There has been no precedent for such a large-scale simultaneous analysis method for plant ceramides.

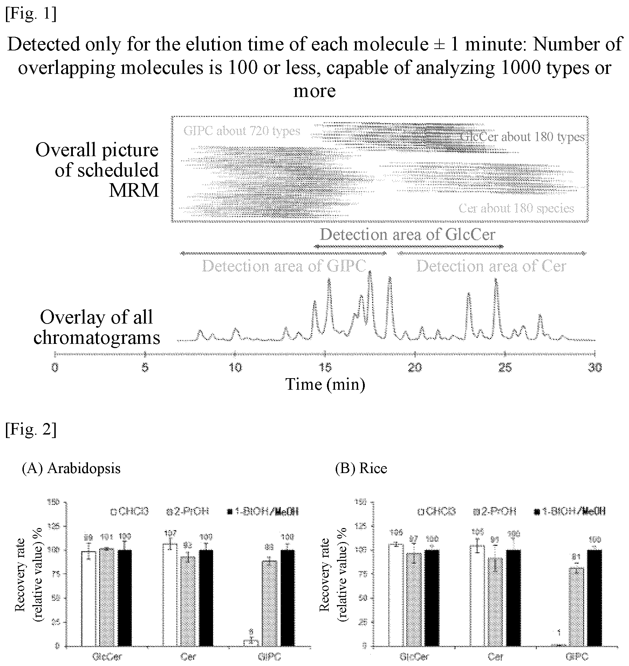

According to the analysis method of the present invention, the abundances of the above three substances can be calculated from the signal intensities obtained, and the total amount of sphingolipids and the composition of each molecular species can be obtained. Figure 1 shows an overall image of scheduled MRM in the analysis method of the present invention and a superposition of the entire chromatograph.

### Analysis method 2 for plant-derived free ceramide

The method for analyzing plant-derived free ceramide according to the present invention includes
a step of preparing the free ceramide produced by the production method of the present invention as an analysis sample;
a step of preparing a control sample by crushing a plant tissue after heating treatment or freeze-drying;
a step of adding a 1-butanol/methanol mixed solution to each of the samples to perform an extraction treatment, and then treating the samples under mild alkaline conditions to decompose the ester lipids;
a step of adding 1-butanol and a strong acid to the sample after the degradation step, separating the sample into two layers under acidic conditions, and recovering 1-butanol in the upper layer to obtain an extract containing free ceramide, glycosylinositol phosphoceramide, and glucosylceramide; and
an analysis step of introducing each of the extracts into a liquid chromatograph-tandem mass spectrometer, separating free ceramide, glycosylinositol phosphoceramide, and glucosylceramide in the extract by liquid chromatography using a reversed-phase column, and performing mass analysis of the separated three substances with the mass spectrometer,
wherein in the analysis step, an MRM measurement is performed that is scheduled to selectively detect only the time around when each of the three substances is eluted from a liquid chromatograph, and the three substances contained in each of the extracts are measured simultaneously, while the amount of free ceramide produced is evaluated from the comparison between the amounts of free ceramide and glycosylinositol phosphoceramide.

### (Step for preparing as sample to be analyzed)

The plant-derived free ceramide produced by the production method of the present invention is prepared as a sample to be analyzed. The details of the production method of the present invention are as described above. Before measurement, it is advisable to inactivate enzymes present intrinsically in the plant tissue in the sample to be analyzed in advance by a heat treatment described below.

### (Step of preparing control sample)

In the step of preparing a control sample, a plant tissue is subjected to a heat treatment or freeze-drying treatment and then crushed to prepare the control sample.

The heat treatment is carried out to inactivate enzymes present in the plant tissue. The treatment temperature is, for example, in the range of 80 to 100°C, preferably 95 to 100°C. The treatment time is, for example, in the range of 1 to 30 minutes, preferably 5 to 20 minutes. The heating method that can be used includes, but is not limited to, a water bath, direct flame heating, and the like.

The freeze-drying treatment is carried out to stop the reaction of enzymes present in the plant tissue by freeze-drying. The freeze-drying method is not particularly limited, but for example, a method in which the plant tissue is placed in a container and freeze-dried using a freeze-dryer can be preferably adopted. Specifically, for example, a method in which the plant tissue is frozen at a temperature of -40°C or lower, then evacuated and dried at a temperature range of -20°C to 30°C can be mentioned.

The crushing means is as already described in the detailed description of the production method of the present invention.

### (Degradation step)

### (Step of obtaining extract)

The above step has already been described in the detailed description of the extraction method of the present invention.

### (Analysis step)

The analysis step involves introducing each of the extracts into a liquid chromatograph-tandem mass spectrometer, separating free ceramide, glycosylinositol phosphoceramide, and glucosylceramide in the extract by liquid chromatograph using a reversed-phase column, mass analyzing the separated three substances with the mass spectrometer, and executing an MRM measurement scheduled to selectively detect only the time around when the three substances are eluted from the liquid chromatograph, thereby simultaneously measuring the three substances contained in each of the extracts, and evaluating the amount of free ceramide produced from the comparison between the amounts of free ceramide and glycosylinositol phosphoceramide.

According to the analysis method of the present invention, the amounts of the three substances present can be calculated from the obtained signal intensity, and the total amount of sphingolipids and the composition of each molecular species can be obtained. According to the analysis method of the present invention, it is possible to clarify, from the results of comparing the sample to be analyzed with the control sample, that GIPC-PLC present in the raw plant tissue decomposes GIPC and directly produces free ceramide. According to the analysis method of the present invention, a plant ceramide library that covers all molecular species that may be present in various plant raw materials, whether known or unknown, is constructed, and detection is limited to the vicinity of the elution time of each molecule, so that more than 1000 molecular species can be analyzed at once. This makes it possible to quantitatively grasp the overall picture of the ceramide metabolic process and rapidly and accurately evaluate the production efficiency of free ceramide.

### EXAMPLES

The features of the present invention will be described more specifically below with reference to examples. Note that the scope of the present invention is not limited to these examples.

### <Production of plant-derived free ceramide>

### 1. GIPC degradation and production of free ceramide in Arabidopsis homogenate

### Example 1 and Control Example 1

1.0 g of raw rosette leaves of Arabidopsis was crushed for about 10 minutes using a mixer (trade name: Waring Blender, model HGBSS, manufactured by WARING) until the tissue mass was sufficiently finely broken down into a uniform suspension, i.e., a so-called homogenate state. The resulting homogenate was left to stand at 25°C for 30 minutes for reaction, and glycosylinositol phosphoceramide derived from Arabidopsis was selectively decomposed into free ceramide by intrinsic phospholipase C to produce free ceramide of Example 1. 1.0 g of raw rosette leaves of Arabidopsis was heat-treated at 95°C for 10 minutes to inactivate intrinsic enzyme, and then crushed using the mixer under the same conditions as in Example 1, and the resulting homogenate was used as Control Example 1. A series of experiments was performed in a laboratory at room temperature of 25°C. The same applies to the following Examples.

### <Preliminary experiment to evaluate the extraction efficiency of plant ceramide-related substances>

(A) Arabidopsis leaves and (B) rice leaves were freeze-dried and then pulverized into powder. Extracts were obtained from 30 mg of each sample using three types of extraction methods: the extraction method of the present invention (method using a 1-butanol/methanol mixture), a method using chloroform (Bligh & Dyer method), and a method using isopropanol (Markham method), and then GlcCer, Cer, and GIPC were quantified by LC-MS/MS. The recovery rate (%) was evaluated as the relative intensity when the detection intensity of the extraction method of the present invention was taken as 100%. The Bligh & Dyer method was carried out based on the method disclosed in the document (E G Bligh, W J DYER: Canadian Journal of Biochemistry and Physiology 1959 August; 37 (8): 911-917). The Markham method was carried out based on the method disclosed in the document (Jennifer E Markham et al: J Biol Chem. 2006 August; 281 (32): 22684-94). As a result, the recovery rates of GlcCer and Cer were similar for both methods, but the recovery rate of GIPC differed greatly depending on the method, with the extraction method of the present invention showing the best value. The results are shown in Fig. 2.

### <Extraction of plant-derived free ceramide>

A 1-butanol/methanol (volume ratio 2:1) mixture was added to Example 1 and Control Example 1, and heated at 80°C for 10 minutes. After cooling, 0.6 volumes (volume) of 1N potassium hydroxide was added, and the mixture was treated at 50°C for 20 minutes to decompose ester lipids. Then, 2 volumes (volume) of 1-butanol and 3 volumes (volume) of 0.4N hydrochloric acid were added to each sample to acidify and separate into two layers. The upper butanol layer was collected, and the solvent was distilled off under reduced pressure to obtain an extract containing free ceramide, GIPC, and glucosylceramide.

### <Analysis of plant-derived free ceramide>

The extract was detected and quantified using a liquid chromatograph-tandem mass spectrometer in multiple reaction monitoring (MRM) mode under the following conditions.

Apparatus: Shimadzu LC-MS8030, ESI, MRM mode (ceramide: [M+H] + > [LCB+H] +, glucosylceramide: [M+H] + > [LCB+H] +, GIPC: [M+H] + > [Cer+H] +), nebulizer flow rate 1.5 L/min, heat block temperature: 400°C, solution A: tetrahydrofuran/methanol/5 mM ammonium formate (150:100:250) + 0.1% formic acid, solution B: tetrahydrofuran/methanol/5 mM ammonium formate (350:100:50) + 0.1% formic acid, gradient conditions: 0 min, A/B = 90:10 > 35 min, A/B = 0:100, column: Shimadzu GLC Sceptor C18 (3 µm, 2.1 x 75 mm), flow rate: 0.2 ml/min

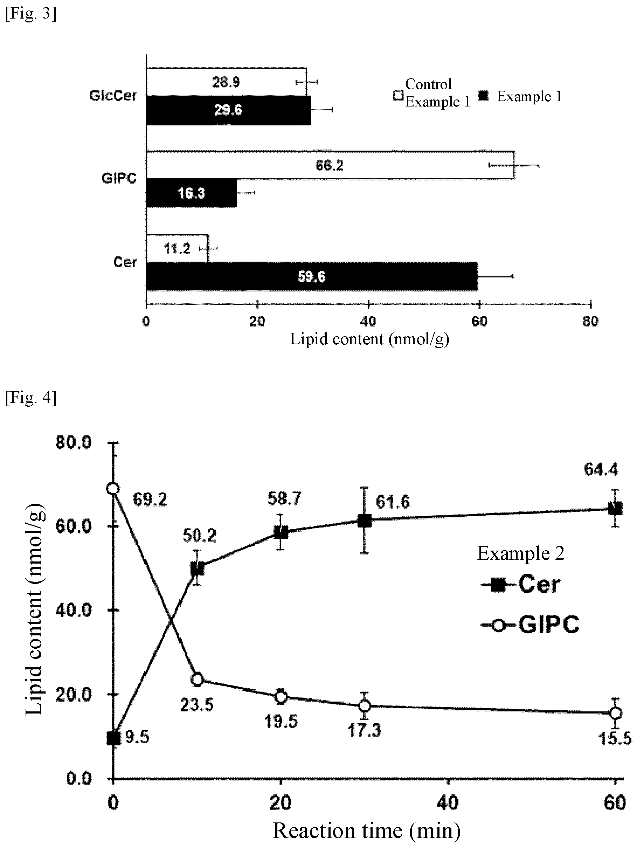

Under the above analytical conditions, the three substances were simultaneously quantitatively analyzed. The amount of free ceramide produced (nmol/g) was evaluated by comparing the free ceramide and GIPC measurements (average values of three measurements) between the examples and the control. The results are shown in Table 3 and Figure 3.

**[Table 3]**

| Unit: nmol/g | | | | |
|---|---|---|---|---|
| | Control Example 1 | | Example 1 | |
| | Baseline | | 30 minutes later | |
| | Average value | Standard deviation | Average value | Standard deviation |
| GlcCer | 28.9 | 1.9 | 29.6 | 3.9 |
| GIPC | 66.2 | 4.5 | 16.3 | 3.2 |
| Cer | 11.2 | 1.6 | 59.6 | 6.4 |

As a result of comparing Example 1 and Control Example 1, in Example 1, most of GIPC (about 80%) was decomposed, and free ceramide was selectively generated (Table 3, Figure 3). The results of Control Example 1 showed that when the plant tissue tissue was heated in advance to inactivate the intrinsic enzyme, degradation of GIPC and generation of free ceramide did not occur. In the prior art (Patent Document 3), it is presumed that when a differentiated plant is crushed and then heated (45°C), glucosylceramide is decomposed by autophagy, but in Example 1, where the reaction was performed under non-heated conditions (25°C), the amount of glucosylceramide hardly changed (see also Example 22 described below). The reason for this is presumed to be that live cells were eliminated by the homogenate produced by crushing.

### Example 2 and Control Example 2

1.0 g of raw rosette leaves of Arabidopsis was crushed using the mixer under the same conditions as in Example 1. The resulting homogenate was allowed to stand at 25°C for 10, 20, 30, and 60 minutes for reaction, and glycosylinositol phosphoceramide derived from Arabidopsis was selectively decomposed into free ceramide by intrinsic phospholipase C to produce free ceramide of Example 2. 1.0 g of raw rosette leaves of Arabidopsis was heat-treated at 95°C for 10 minutes to inactivate the intrinsic enzyme, and then crushed in the same manner using the mixer, and the resulting homogenate was used as Control Example 2.

For Example 2 and Control Example 2, simultaneous quantitative analysis of free ceramide was performed in the same manner as in Example 1 and Control Example 1, and the amount of free ceramide produced (nmol/g) was evaluated from the comparison between the amount of free ceramide and the amount of glycosylinositol phosphoceramide. The results are shown in Table 4 and Figure 4.

**[Table 4]**

| Unit: nmol/g | | | | | | | | | | |
|---|---|---|---|---|---|---|---|---|---|---|
| | Control Example 1 | | Example 2 | | | | | | | |
| | Baseline | | 10 minutes later | | 20 minutes later | | 30 minutes later | | 60 minutes later | |
| | Average value | Standard deviation | Average value | Standard deviation | Average value | Standard deviation | Average value | Standard deviation | Average value | Standard deviation |
| GIPC | 69.2 | 7.8 | 23.5 | 1.7 | 19.5 | 1.7 | 17.3 | 3.2 | 15.5 | 3.6 |
| Cer | 9.5 | 2.3 | 50.2 | 4.1 | 58.7 | 4.1 | 61.6 | 7.8 | 64.4 | 4.4 |

Comparison of Example 2 with Control Example 2 showed that in Example 2, rapid degradation of GIPC occurred about 10 minutes after crushing, and the reaction proceeded slowly thereafter (Table 4, Fig. 4).

### 2. GIPC degradation and production of free ceramide in various plant bodies Examples 3 to 12 and Control Examples 3 to 12

Each plant tissue-derived free ceramide was produced in the same manner as in Example 1, except that 1.0 g of each raw plant tissue shown in Table 5 was used. Control Examples 3 to 12 were obtained in the same manner as in Control Example 1. The standing time (reaction time) of the homogenate in each Example was 1 hour or 24 hours.

**[Table 5]**

| Examples and Control Examples | Plant tissue | | |
|---|---|---|---|
| | Name | Family name | Area of use |
| 3 | Arabidopsis | Brassicaceae | Rosette leaves |
| 4 | Radish | Brassicaceae | Edible portion |
| 5 | Japanese mustard spinach | Brassicaceae | Edible portion |
| 6 | Broccoli | Brassicaceae | Edible portion |
| 7 | Bok choy | Brassicaceae | Edible portion |
| 8 | Mizuna | Brassicaceae | Edible portion |
| 9 | Carrot | Apiaceae | Edible portion |
| 10 | Bean sprouts | Leguminous | Edible portion |
| 11 | Basil | Lamiaceae | Edible portion |
| 12 | Green onion | Amaryllidaceae | Edible portion |

For Examples 3 to 12 and Control Examples 3 to 12, the lipid composition (mol %) was evaluated by simultaneous quantitative analysis of free ceramide in the same manner as in Example 1 and Control Example 1. The results are shown in FIG.

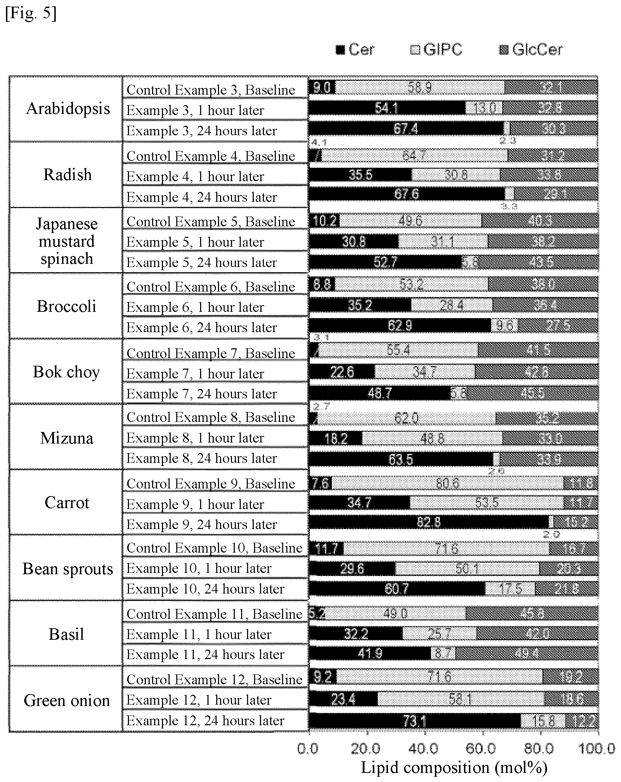

As shown in Figure 5, GIPC was decomposed by GIPC-PLC present in various plant bodies, and an increase in free ceramide was observed. Arabidopsis had the highest GIPC degradation activity after 1 hour. Over time, the degradation of GIPC progressed, and the amount of free ceramide increased in all plant species. On the other hand, the amount of glucosylceramide hardly changed over time, indicating that the reactions observed in Examples 3 to 12 were GIPC-specific degradation enzyme reactions that are completely different from autophagy shown in the prior art (Patent Document 3).

Furthermore, the distribution of fatty acid chain lengths in the free ceramide composition (mol %) 24 hours after GIPC degradation was evaluated for Examples 3 to 5. The results are shown in Table 6.

**[Table 6]**

| Fatty acid chain length | Free ceramide composition after GIPC degradation (mol%) | | |
|---|---|---|---|
| | Arabidopsis (Example 3) | Radish (Example 4) | Japanese mustard spinach(Example 5) |
| 16 | 11.6 | 3.7 | 10.6 |
| 18 | 0.3 | 0.3 | 0.8 |
| 20 | 1.3 | 1.3 | 1.5 |
| 22 | 10.9 | 19.3 | 16.4 |
| 24 | 65.8 | 68.7 | 57.7 |
| 26 | 10.2 | 6.2 | 12.8 |

As shown in Table 6, the plant-derived free ceramide obtained by the production method of the present invention was found to contain the same main component as the human type, C24, although there was some variation depending on the plant species.

### 3. Degradation of GIPC in heterologous plant tissue by Arabidopsis homogenate Example 13 and Control Example 13

0.5 g of raw rosette leaves of Arabidopsis was mixed with 1.0 g of carrot that had been heat-treated at 95°C for 10 minutes to inactivate intrinsic enzyme, and the mixture was crushed for 3 minutes using a mixer (trade name: Waring Blender, model HGBSS, manufactured by WARING) until the tissue mass was sufficiently finely broken down into a uniform suspension, i.e., a homogenate state. The resulting homogenate was allowed to stand at 25°C for 30 minutes, 1 hour, 3 hours, 6 hours, and 24 hours for reaction to produce free ceramide. 1.0 g of raw rosette leaves of Arabidopsis was heat-treated at 95°C for 10 minutes to inactivate intrinsic enzyme, and then crushed using the mixer, and the resulting homogenate was used as Control Example 13.

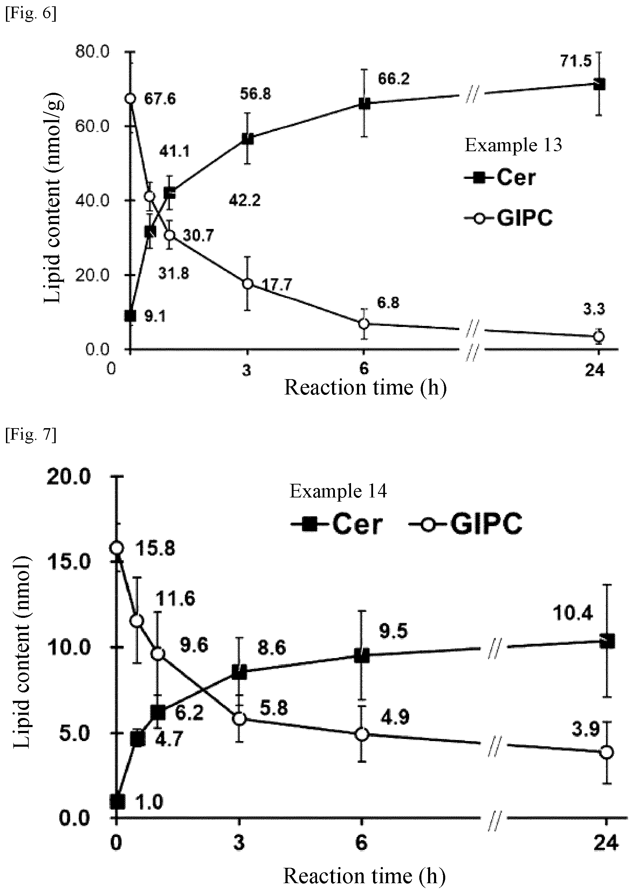

For Example 13 and Control Example 13, simultaneous quantitative analysis of free ceramide was performed to evaluate the amount of free ceramide produced (nmol/g) in the same manner as in Example 1 and Control Example 1. The composition of each plant tissue sample is shown in Table 7, and the changes in free ceramide and GIPC over time are shown in Table 8 and Figure 6. The amounts of free ceramide and GIPC at the baseline in Figure 6 are the values for Arabidopsis in Control Example 13.

**[Table 7]**

| Sample composition | | Arabidopsis | Carrots (heat inactivated) |
|---|---|---|---|
| Fat content (mmol/g) | GIPC | 66.2 | 74.6 |
| | Cer | 11.2 | 7.6 |
| Amount used | | 0.5 g | 1.0 g |
| Mixing weight ratio | | 1 | 1 |

**[Table 8]**

| Unit: nmol/g | | | | | | | | | | | | |
|---|---|---|---|---|---|---|---|---|---|---|---|---|
| | Control Example 13 | | Example 13 | | | | | | | | | |
| | Baseline | | 30 minutes later | | 1 hour later | | 3 hours later | | 6 hours later | | 24 hours later | |
| | Average value | Standard deviation | Average value | Standard deviation | Average value | Standard deviation | Average value | Standard deviation | Average value | Standard deviation | Average value | Standard deviation |
| GIPC | 67.6 | 9.3 | 41.1 | 3.8 | 30.7 | 3.8 | 17.7 | 7.2 | 6.8 | 4.1 | 3.3 | 2.0 |
| Cer | 9.1 | 2.8 | 31.8 | 4.6 | 42.2 | 4.6 | 56.8 | 6.9 | 66.2 | 9.1 | 71.5 | 8.3 |

In Example 13, as the standing time (reaction time) increased, the degradation of GIPC in carrot progressed and the amount of GIPC converted to free ceramide also increased (Fig. 6 ). The conversion efficiency from GIPC to free ceramide after 3, 6, and 24 hours was about 75%, about 90%, and about 95%, respectively.

### 4. Degradation of soybean-derived GIPC by Arabidopsis homogenate

### Example 14 and Control Example 14

10 nmol of GIPC extracted from soybeans was dried under a nitrogen stream, and the homogenate equivalent to 0.1 g of raw rosette leaves of Arabidopsis was added, and suspended by ultrasonic treatment for 30 seconds at 25°C using an ultrasonic treatment device (product name US-2KS, manufactured by SND Co., Ltd.). The resulting suspension was allowed to stand at 25°C for 30 minutes, 1 hour, 3 hours, 6 hours, and 24 hours for reaction to produce free ceramide.

0.1 g of raw rosette leaves of Arabidopsis was heat-treated at 95°C for 10 minutes to inactivate intrinsic enzyme, and then crushed in the mixer, and the resulting homogenate was used as Control Example 14.

For Example 14 and Control Example 14, simultaneous quantitative analysis of free ceramide was performed in the same manner as in Example 1 and Control Example 1, and the amount of free ceramide produced (nmol) was evaluated. The composition of each sample is shown in Table 9, and the changes in free ceramide and GIPC over time are shown in Table 10 and Fig. 7. The amounts of free ceramide and GIPC at the baseline in Fig. 7 are the values for Arabidopsis in Control Example 14.

**[Table 9]**

| Sample composition | | Arabidopsis | Soybean-derived GIPC |
|---|---|---|---|
| Fat content (mmol/g) | Free ceramide | 1.1 | 0 |
| | GIPC | 6.6 | 10 |

**[Table 10]**

| Unit: nmol | | | | | | | | | | | | |
|---|---|---|---|---|---|---|---|---|---|---|---|---|
| | Control Example 14 | | Example 14 | | | | | | | | | |
| | Baseline | | 30 minutes later | | 1 hour later | | 3 hours later | | 6 hours later | | 24 hours later | |
| | Average value | Standard deviation | Average value | Standard deviation | Average value | Standard deviation | Average value | Standard deviation | Average value | Standard deviation | Average value | Standard deviation |
| GIPC | 15.8 | 1.4 | 11.6 | 2.5 | 9.6 | 2.4 | 5.8 | 1.4 | 4.9 | 1.6 | 3.9 | 1.8 |
| Cer | 1.0 | 0.1 | 4.7 | 0.5 | 6.2 | 1.0 | 8.6 | 2.0 | 9.5 | 2.6 | 10.4 | 3.3 |

In Example 14, the degradation of soybean-derived GIPC progressed with increasing standing time, and the amount of ceramide converted to free ceramide also increased (Fig. 7). This shows that free ceramide can be produced from GIPC extracted from plant tissue tissues.

### 5. Production of free ceramide by mixing GIPC-PLC-containing sample with heterologous plant tissue

### Examples 15 to 20 and Control Example 15

1.0 g of carrot, which had been heat-treated at 95°C for 10 minutes to inactivate the intrinsic enzyme, was mixed with 0.01 g (Example 15), 0.05 g (Example 16), 0.1 g (Example 17), 0.25 g (Example 18), 0.5 g (Example 19), and 1.0 g (Example 20) of raw rosette leaves of Arabidopsis, and crushed in the mixer until homogenized. The resulting homogenate was allowed to stand at 25°C for 3 hours to react, producing free ceramide. 1.0 g of carrot (edible part) was heat-treated at 95°C for 10 minutes to inactivate the intrinsic enzyme, and then crushed in the mixer, and the resulting homogenate was used as Control Example 15.

### Comparative Examples 1 and 2

After freezing the enoki mushrooms overnight, they were allowed to self-digest in a thermostatic chamber at 25°C, and after 24 hours, EDTA was added and the mushrooms were crushed in a food processor. The homogenate was separated into solid and liquid, and the liquid portion was prepared as enoki mushroom autolysis liquid. 1.0 g of carrot, which had been heat-treated at 95°C for 10 minutes to inactivate the intrinsic enzyme, was mixed with the enoki mushroom autolysis liquid equivalent to 0.25 g (Comparative Example 1) or 1.0 g (Comparative Example 2) of enoki mushroom weight, and crushed in the mixer. The resultant homogenate was left to stand at 25°C for 3 hours to produce free ceramide.

For Examples 15 to 20, Control Example 15, and Comparative Examples 1 and 2, free ceramide was analyzed in the same manner as in Example 1 and Control Example 1, and the ratio (%) of GIPC to free ceramide was determined. The results are shown in FIG.

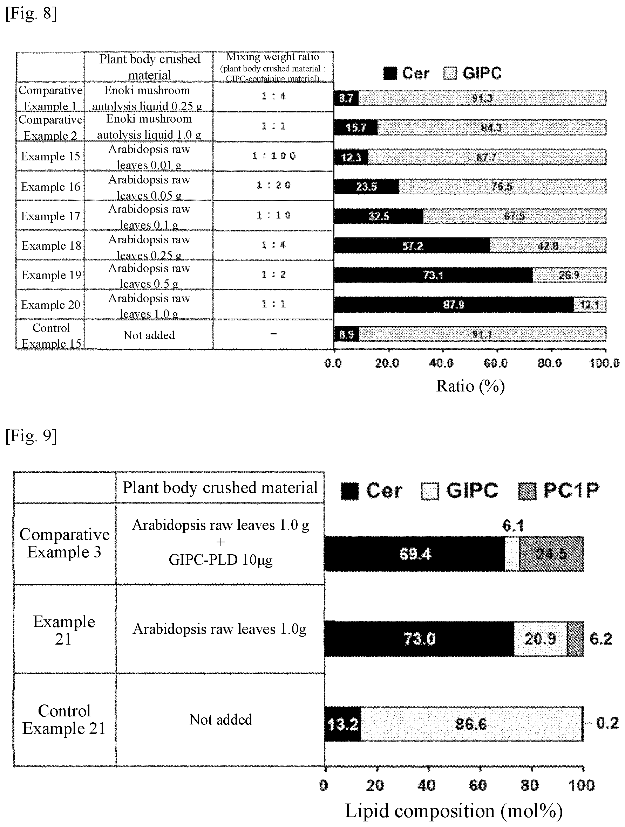

As shown in Figure 8, the conversion efficiency from GIPC to free ceramide correlated with the amount of plant tissue homogenate (enzyme-containing sample) added. The mixing weight ratio of plant tissue homogenate to GIPC-containing material for producing free ceramide in a short time is preferably 1:1 to 1:4, but even if the mixing ratio of plant tissue homogenate is low, the conversion efficiency can be increased by extending the reaction time. The GIPC degradation rate of the enoki mushroom autolysis liquid equivalent to 1.0 g of enoki mushroom in Comparative Example 2 was only about 7%, whereas the GIPC degradation rate when 0.05 g of Arabidopsis was used in Example 16 was about 15%. When comparing the conversion ratios of each degradation enzyme-containing sample equivalent to the same weight, the difference was about 6.33 times between Example 18 and Comparative Example 1, which are equivalent to 0.25 g, and about 5.5 times between Example 20 and Comparative Example 2, which are equivalent to 1.0 g. From the above, it was shown that the Arabidopsis homogenate of Examples 15 to 20 have a higher GIPC activity than the Enoki autolysis liquid of Comparative Examples 1 and 2. In addition, the Arabidopsis homogenate of Examples 15 to 20 can be prepared by a simple operation of simply crushing under non-heated conditions (25°C), whereas the Enoki autolysis liquid of Comparative Examples 1 and 2 requires a step of freezing, then thawing and autolyzing, and an extraction step. From this point of view, the advantages of Examples 15 to 20 over Comparative Examples 1 and 2, in that plant-derived free ceramide can be produced simply and efficiently, were shown.

### 6. Verification of differences from known GIPC degradation pathways (GIPC-PLD pathway)

### Example 21 and Control Example 21

1.0 g of raw rosette leaves of Arabidopsis was crushed using the mixer until it became homogenized. The resulting homogenate was allowed to stand at 25°C for 60 minutes to react and produce free ceramide. 1.0 g of raw rosette leaves of Arabidopsis was heat-treated at 95°C for 10 minutes to inactivate intrinsic enzyme, and then crushed using the mixer, and the resulting homogenate was used as Control Example 21.

### Comparative Example 3

The GIPC-PLD gene derived from Arabidopsis was introduced into the E. coli expression vector pBAD-DEST49 (Thermo Fisher Scientific) to obtain a recombinant GIPC-PLD enzyme derived from E. coli. 1.0 g of raw rosette leaves of Arabidopsis was mixed with 10 µg of the GIPC-PLD enzyme derived from E. coli and crushed using the mixer until homogenized. The resulting homogenate was allowed to stand at 25°C for 60 minutes to react, producing free ceramide.

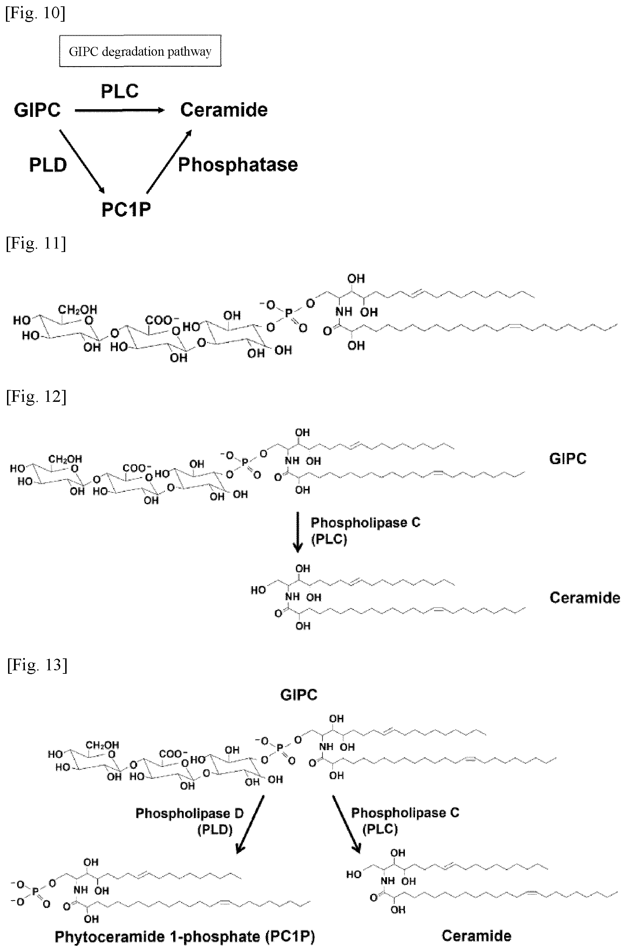

For Example 21, Control Example 21, and Comparative Example 3, free ceramide analysis was performed and the amount of free ceramide produced (mol %) was evaluated in the same manner as in Example 1 and Control Example 1. The results are shown in Figure 9, and the mechanism of the presumed GIPC degradation pathway is shown in Figure 10.

As shown in Fig. 9, when GIPC-PLD was added to Arabidopsis homogenate, GIPC decreased and phytoceramide-1-phosphate (PC1P) increased, but free ceramide did not increase (Comparative Example 3). This result suggests that the production of free ceramide by the plant tissue homogenate is via the PLC pathway, independent of the PLD pathway via PC1P (Fig. 10). A previous report (Non-Patent Document 1) suggested that GIPC-PLD, which specifically hydrolyzes GIPC to PC1P, was found in cabbage, and that PC1P was likely converted to ceramide by alkaline phosphatase in the small intestine. However, as shown in Fig. 10, if there is an intrinsic pathway for producing free ceramide via the PLD pathway as a degradation pathway for GIPC, the amount of free ceramide should increase even by adding PLD from the outside. However, the amount of free ceramide did not increase, but rather slightly decreased. This result suggested that the PLC pathway contributed greatly to the degradation of GIPC, while the PLD pathway contributed only slightly or almost nothing.

GIPC has a structure of ceramide-phosphate-inositol-oligosaccharide (Figure 11), and free ceramide is generated by cleaving the phosphate ester bond between this ceramide and phosphate (Figure 12). An enzyme called GIPC-PLC cleaves this part. As is clear from the analysis results of free ceramide described above, the decrease in GIPC and the increase in free ceramide are roughly equal, and as shown in Comparative Example 3, the addition of GIPC-PLD to the reaction system does not increase free ceramide, indicating that the increased free ceramide is produced directly from GIPC and that PLC activity is present (Figure 13).

### 7. Difference in the amount of free ceramide produced due to differences in temperature conditions in degradation process

### Example 22 and Control Example 22

1.0 g of raw rosette leaves of Arabidopsis was crushed using the mixer until it became homogenized. The resulting homogenate was allowed to stand at 25°C for 3 hours for reaction to produce free ceramide. 1.0 g of raw rosette leaves of Arabidopsis was heat-treated at 95°C for 10 minutes to inactivate intrinsic enzyme, and then crushed using the mixer, and the resulting homogenate was used as Control Example 22.

### Comparative Example 4

1.0 g of raw rosette leaves of Arabidopsis was crushed using the mixer until it became homogenous. The resulting homogenate was allowed to stand at 45°C for 3 hours to produce free ceramide.

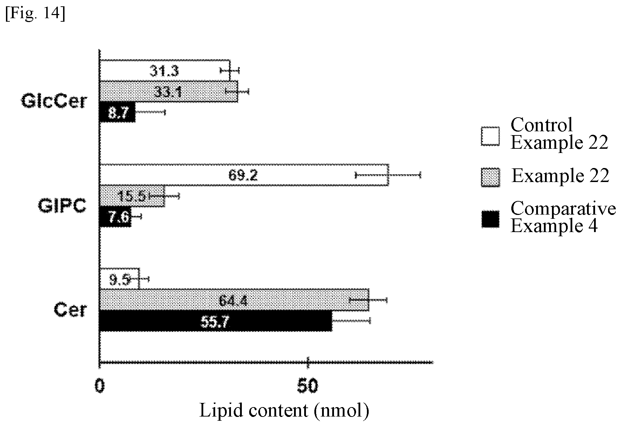

For Example 22, Control Example 22, and Comparative Example 4, free ceramide analysis was performed in the same manner as in Example 1 and Control Example 1, and the amount of free ceramide produced (nmol/g) was evaluated. The results are shown in Table 11 and Figure 14.

**[Table 11]**

| Unit: nmol/g | | | | | | |
|---|---|---|---|---|---|---|
| | Control Example 22 | | Example 22 | | Comparative Example 4 | |
| | 25°C, baseline | | 25°C, 3 hours later | | 45°C, 3 hours later | |
| | Baseline | Standard deviation | Baseline | Standard deviation | Baseline | Standard deviation |
| GlcCer | 31.3 | 2.2 | 33.1 | 2.7 | 8.7 | 6.9 |
| GIPC | 69.2 | 7.8 | 15.5 | 3.6 | 7.6 | 2.3 |
| Cer | 9.5 | 2.3 | 64.4 | 4.4 | 55.7 | 9.1 |

As shown in Figure 14, comparing 25°C (Example 22) and 45°C (Comparative Example 4), GIPC was actively decomposed at the high temperature condition of 45°C, but while the degradation rates of GIPC and GlcCer increased, the amount of free ceramide produced did not increase compared to 25°C, and tended to be smaller. From this, it is speculated that various degradation reactions, including the degradation reaction of free ceramide, occur under the high temperature condition of 45°C. On the other hand, at 25°C, only the GIPC degradation reaction occurs, and degradation reactions of free ceramide and GlcCer do not occur. From the above, it was shown that the temperature conditions suitable for free ceramide production by GIPC-PLC are in the range of 20 to 30°C.

The inventions described in the claims originally attached to the application of the earlier application on which priority is based are listed below. The claim numbers in the appendix are the same as those in the claims originally attached to the application of the earlier application.

### <Claim 1>

A method for producing plant-derived free ceramide, comprising
an intrinsic enzyme activation step of crushing a plant tissue to obtain a plant tissue homogenate in which phospholipase C that has glycosylinositol phosphoceramide as a substrate intrinsic in the plant tissue is activated; and
a degradation step of reacting the plant tissue homogenate with a plant-derived glycosylinositol phosphoceramide-containing material to decompose the glycosylinositol phosphoceramide into free ceramide.

### <Claim 2>

The method for producing plant-derived free ceramide according to claim 1, wherein the glycosylinositol phosphoceramide-containing material is any one of the following or a combination thereof:
(a) glycosylinositol phosphoceramide contained in the plant tissue,
(b) glycosylinositol phosphoceramide contained in a heterologous plant tissue,
(c) glycosylinositol phosphoceramide, which is an extract extracted from the plant tissue, and
(d) glycosylinositol phosphoceramide, which is an extract extracted from a heterologous plant tissue.

### <Claim 3>

The method for producing plant-derived free ceramide according to claim 1, wherein the mixing weight ratio of the plant tissue homogenate to the glycosylinositol phosphoceramide-containing material in the degradation step is 1:0 to 1:150.

### <Claim 4>

The method for producing plant-derived free ceramide according to claim 1, wherein the reaction temperature in the degradation step is within the range of 20 to 30°C.

### <Claim 5>

A method for analyzing plant-derived free ceramide, comprising
a step of preparing the free ceramide produced by the production method according to claim 1 as an analysis sample;
a step of preparing a control sample by crushing a plant tissue after heating treatment or freeze-drying;
a step of adding a solvent to each of the samples to extract an extract containing free ceramide, glycosylinositol phosphoceramide, and glucosylceramide; and
an analysis step of introducing each of the extracts into a liquid chromatograph-tandem mass spectrometer, separating free ceramide, glycosylinositol phosphoceramide, and glucosylceramide in the extract by liquid chromatography using a reversed-phase column, and performing mass analysis of the separated three substances with the mass spectrometer,
wherein the extraction step includes
a step of adding a 1-butanol/methanol (volume ratio 2:1) mixture to each of the samples, treating the sample at 75°C or higher for 10 minutes or more, then adding 0.3 to 1 volume of 1N potassium hydroxide, and treating the sample at 45 to 70°C for 10 minutes or more to decompose ester lipids, and a step of adding 1.5 to 3 volumes of 1-butanol and 1.5 to 5 volumes of 0.4 N hydrochloric acid to each of the samples after the degradation step to acidify the samples and separate them into two layers, and recovering 1-butanol in the upper layer, and
wherein in the analysis step, an MRM measurement is performed scheduled to selectively detect only the time around when each of the three substances is eluted from a liquid chromatograph, and the three substances contained in each sample are measured simultaneously, while the amount of free ceramide produced is evaluated by comparing the amount of free ceramide with that of glycosylinositol phosphoceramide.

### Industrial Applicability

The production method of the present invention is promising in the following two respects. The first is the improvement of the quality of existing free ceramide raw materials. For example, free ceramide can be produced from raw products, processed products, residues, etc. of plant raw materials that have already been used in food and cosmetics. The yield of the free ceramide is equal to or higher than that of existing plant-derived glucosylceramide. The production method of the present invention can increase the functionality and added value of plant-derived free ceramide, contributing to higher quality. The second is the resource utilization of plant waste. This technique is versatile and can utilize plant waste from farmers, fruit and vegetable markets, and the food industry as a ceramide raw material. Agricultural waste that is currently passively disposed of by plowing into the soil, etc., can be actively utilized to contribute to the SDGs. In addition, it is possible to return profits to farmers and reduce the cost of raw materials. The production method of the present invention is also useful for producing plant-derived free ceramide that is used as a component or raw material for foods and beverages including functional foods, cosmetics, pharmaceuticals, etc. The extraction method of the present invention is useful because it can recover nearly 100% of all molecular species of ceramide-related substances. The analysis method of the present invention is useful because it can simultaneously analyze more than 1000 molecular species of plant-derived free ceramide, GIPC, and glucosylceramide.

## Claims

1. A method for producing plant-derived free ceramide, comprising
an enzyme activation step by destroying cells of a plant tissue excluding calli to obtain a plant tissue homogenate in which an endogenous activated phospholipase C that hydrolyzes glycosylinositol phosphoceramide as a substrate is included; and
a degradation step of the glycosylinositol phosphoceramide by reacting the plant tissue homogenate together with same and /or another plant-derived glycosylinositol phosphoceramide-containing material at a temperature range of 20 to 30°C to selectively hydrolyze the glycosylinositol phosphoceramide into free ceramide by the endogenous activated phospholipase C.

2. The method for producing plant-derived free ceramide according to claim 1,
wherein the plant-derived glycosylinositol phosphoceramide-containing material is any one of the following or a combination thereof:
(a) the plant tissue containing glycosylinositol phosphoceramide,
(b) a different plant tissue containing glycosylinositol phosphoceramide,
(c) glycosylinositol phosphoceramide extracted from the plant tissue containing the phospholipase C, and
(d) glycosylinositol phosphoceramide extracted from a tissue of other plant species.

3. The method for producing plant-derived free ceramide according to claim 1, wherein the mixing weight ratio of the plant tissue homogenate to the plant-derived glycosylinositol phosphoceramide-containing material in the degradation step is 1:0 to 1:150.

4. Plant-derived free ceramide obtained by the production method according to any one of claims 1 to 3.

5. A food or drink comprising the plant-derived free ceramide according to claim 4.

6. A cosmetic comprising the plant-derived free ceramide according to claim 4.

7. A pharmaceutical comprising the plant-derived free ceramide according to claim 4.

8. A method for extracting the plant-derived free ceramide obtained by the production method according to claim 1, comprising
an alkaline treatment step for decomposing ester lipids by adding a 1-butanol/methanol mixture to a sample for lipid extraction, and then treating the sample under mild alkaline conditions ; and
a step for obtaining an extract by adding 1-butanol and a strong acid to the sample after the alkaline treatment step, separating the sample into two layers under acidic conditions, and recovering the upper 1-butanol-rich layer to obtain an extract containing free ceramide, glycosylinositol phosphoceramide, and glucosylceramide.

9. A method for analyzing the plant-derived free ceramide obtained by the production method according to claim 1, comprising
an alkaline treatment step for decomposing ester lipids by adding a 1-butanol/methanol mixture to a sample for lipid extraction treatment, and then treating the sample under mild alkaline conditions;
a step for obtaining an extract by adding 1-butanol and a strong acid to the sample after the alkaline treatment step, separating the sample into two layers under acidic conditions, and recovering the upper 1-butanol-rich layer to obtain an extract containing free ceramide, glycosylinositol phosphoceramide, and glucosylceramide; and
an analysis step of introducing the extract into a liquid chromatograph-tandem mass spectrometer, separating molecular species of free ceramide, glycosylinositol phosphoceramide, and glucosylceramide in the extract by liquid chromatography using a reversed-phase column, and performing mass spectrometry on the separated substances with the mass spectrometer,
wherein in the analysis step, an MRM measurement is performed for selective detection and quantification of the substances, which is scheduled to monitor around chromatographic elution time of each substance, allowing simultaneous quantification of the whole species of free ceramide, glycosylinositol phosphoceramide, and glucosylceramide contained in the extract.

10. A method for analyzing plant-derived free ceramide, comprising
a step of preparing the free ceramide produced by the production method according to claim 1 as an analyte
a step of preparing a control sample by homogenizing a plant tissue after heating or freeze-drying;
a step of adding a 1-butanol/methanol mixture to each of the samples for lipid extraction, and then treating the samples under mild alkaline conditions to decompose ester lipids;
a step of adding 1-butanol and a strong acid to the sample after the alkaline treatment, separating the sample into two layers under acidic conditions, and recovering the upper 1-butanol-rich layer to obtain an extract containing free ceramide, glycosylinositol phosphoceramide, and glucosylceramide; and
an analysis step of introducing each of the extracts into a liquid chromatograph-tandem mass spectrometer, separating molecular species of free ceramide, glycosylinositol phosphoceramide, and glucosylceramide in the extract by liquid chromatography using a reversed-phase column, and performing mass analysis of the separated three substances with the mass spectrometer,
wherein in the analysis step, an MRM measurement is performed for selective detection and quantification of the substances, which is scheduled to monitor around chromatographic elution time of each substance, and the whole species of ceramide, glycosylinositol phosphoceramide contained in the extracts are measured simultaneously, and then the amount of free ceramide produced is evaluated by the increased amount of free ceramide and decreased amount of glycosylinositol phosphoceramide.
